(19) Europäisches Patentamt
European Patent Office
Office européen des brevets

(11) Veröffentlichungsnummer: **0 253 175 B1**

# (12) EUROPÄISCHE PATENTSCHRIFT

(45) Veröffentlichungstag der Patentschrift: **30.12.92**

(51) Int. Cl.[5]: **C07D 271/08**, A01N 43/82, C07C 211/00, C07C 205/00

(21) Anmeldenummer: **87109229.2**

(22) Anmeldetag: **26.06.87**

## (54) Substituierte Furazane.

(30) Priorität: **08.07.86 DE 3622862**

(43) Veröffentlichungstag der Anmeldung:
**20.01.88 Patentblatt 88/03**

(45) Bekanntmachung des Hinweises auf die Patenterteilung:
**30.12.92 Patentblatt 92/53**

(84) Benannte Vertragsstaaten:
**AT BE CH DE FR GB IT LI NL**

(56) Entgegenhaltungen:
**EP-A- 0 002 620**
**EP-A- 0 132 680**

(73) Patentinhaber: **BAYER AG**

**W-5090 Leverkusen 1 Bayerwerk(DE)**

(72) Erfinder: **Sirrenberg, Wilhelm, Dr.**
**Wuppertaler Strasse 21**
**W-4322 Sprockhövel(DE)**
Erfinder: **Marhold, Albrecht, Dr.**
**Carl-Duisberg-Strasse 329**
**W-5090 Leverkusen 1(DE)**
Erfinder: **Steffens, Robert, Dr.**
**Roggendorfstrasse 63**
**W-5000 Köln 80(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Furazane, Verfahren zu ihrer Herstellung und ihre Verwendung als Schädlingsbekämpfungsmittel, insbesondere als Insektizide und Akarizide.

Es ist bisher bekannt, daß substituierte Furazane, wie z. B. 1-(4-tert.-Butyl-phenyl)-3-[4-(4-chlorphenyl)-1,2,5-oxadiazol-3-yl)-harnstoff, 1-(4-Trifluormethyl-phenyl)-3-(4-phenyl-1,2,5-oxadiazol-3-yl)-harnstoff und 1-(4-Bromphenyl)-3-(4-phenyl-1,2,5-oxadiazol-3-yl)-harnstoff insektizide und akarizide Eigenschaften aufweisen (vergl. EP-A 132 680).

Es wurden nun die neuen substituierten Furazane der Formel (I)

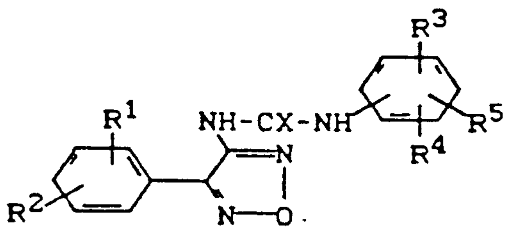

(I)

in welcher

$R^1$ und $R^2$ gleich oder verschieden sind und für Wasserstoff, Halogen, $C_1$-$C_6$-Alkyl, $C_1$-$C_6$-Alkoxy, $C_1$-$C_6$-Alkylthio, Halogen-$C_1$-$C_6$-alkyl, Halogen-$C_1$-$C_6$-alkoxy, Halogen-$C_1$-$C_6$-alkylthio oder für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy und/oder Halogen-$C_1$-$C_4$-alkylthio substituiertes Aryloxy mit 6 bis 10 Kohlenstoffatomen im Arylteil stehen, oder

$R^1$ und $R^2$ gemeinsam für einen gegebenenfalls durch Halogen und/oder $C_1$-$C_2$-Alkyl substituierten $C_1$-$C_3$-Alkylen-Rest, welcher durch 1 oder 2 Sauerstoffatome unterbrochen wird oder über 1 oder 2 Sauerstoffatome an den Phenylring gebunden ist, stehen,

$R^3$ und $R^4$ gleich oder verschieden sind und für Wasserstoff, Halogen, $C_1$-$C_6$-Alkyl, $C_1$-$C_6$-Alkoxy, Halogen-$C_1$-$C_6$-alkyl oder Halogen-$C_1$-$C_4$-alkoxy stehen,

$R^5$ für gegebenenfalls durch Halogen und/oder $C_1$-$C_2$-Alkyl substituiertes Cycloalkyl mit 3 bis 8 Kohlenstoffatomen steht und

X für Sauerstoff oder Schwefel steht,

gefunden.

Weiterhin wurde gefunden, daß man die neuen substituierten Furazane der Formel (I) erhält, indem man

a) 3-Amino-1,2,5-oxadiazole der Formel (II)

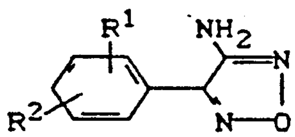

(II)

in welcher

$R^1$ und $R^2$ die oben angegebenen Bedeutungen haben,

mit Iso(thio)cyanaten der Formel (III)

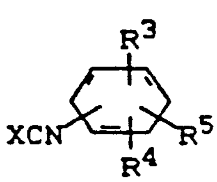

(III)

in welcher

X, $R^3$, $R^4$ und $R^5$ die oben angegebenen Bedeutungen haben,
gegebenenfalls in Gegenwart von Katalysatoren und gegebenenfalls in Gegenwart von Verdünnungsmitteln bei Temperaturen zwischen 20 und 180 °C umsetzt, oder
b) 3-Iso(thio)cyanato-1,2,5-oxadiazole der Formel (IV)

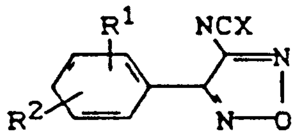

(IV)

in welcher
X, $R^1$ und $R^2$ die oben angegebenen Bedeutungen haben,
mit Anilinen der Formel (V)

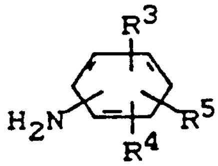

(V)

in welcher
$R^3$, $R^4$ und $R^5$ die oben angegebenen Bedeutungen haben,
gegebenenfalls in Gegenwart von Katalysatoren und gegebenenfalls in Gegenwart von Verdünnungsmitteln bei Temperaturen zwischen 20 und 200 °C umsetzt.

Die Verbindungen der Formel (I) können in verschiedenen geometrischen und optischen Isomerenformen vorliegen, je nach Anordnung der Substituenten die an den Cycloalkyl-Rest gebunden sind; vorzugsweise fallen sie in einem wechselnden Isomerenverhältnis an. Die vorliegende Erfindung betrifft sowohl die einzelnen Isomeren als auch die Isomerengemische.

Alkyl $R^1$, $R^2$, $R^3$ und $R^4$ ist geradkettig oder verzweigt und enthält 1 bis 6, insbesondere 1 bis 4 Kohlenstoffatome.

Beispielhaft seien Methyl, Ethyl, n-Propyl, i-Propyl, n-Butyl, i-Butyl, sec.-Butyl und tert.-Butyl (vorzugsweise Methyl) genannt.

Alkoxy $R^1$, $R^2$, $R^3$ und $R^4$ enthalten im Alkylteil geradkettiges oder verzweigtes Alkyl mit 1 bis 6, insbesondere 1 bis 4 Kohlenstoffatomen.

Beispielhaft seien Methoxy, Ethoxy, n-Propoxy, i-Propoxy, n-Butoxy, i-Butoxy, sec.-Butoxy und tert.-Butoxy (vorzugsweise Methoxy) genannt.

Alkylthio $R^1$ und $R^2$ enthalten im Alkylteil geradkettiges oder verzweigtes Alkyl mit 1 bis 6, insbesondere 1 bis 4 Kohlenstoffatomen.
Beispielhaft seien Methyl-, Ethyl-, n-Propyl-, i-Propyl, n-Butyl-, i-Butyl-, sec.-Butyl- und tert.-Butylthio (vorzugsweise Methylthio) genannt.

Halogenalkyl und Halogenalkoxy $R^1$, $R^2$, $R^3$ und $R^4$ und Halogenalkylthio $R^1$ und $R^2$ enthalten im Alkylteil geradkettiges oder verzweigtes Alkyl mit 1 bis 6, besonders bevorzugt 1 bis 4, insbesondere 1 bis 2 Kohlenstoffatomen und vorzugsweise 1 bis 6, insbesondere 1 bis 4 Halogenatome, wobei die Halogenatome gleich oder verschieden sind und als Halogenatome vorzugsweise Fluor, Chlor oder Brom, insbesondere Fluor, stehen. Beispielhaft seien genannt Trifluormethyl, Chlordifluormethyl, Difluormethyl, Trifluormethylthio, Chlordifluormethylthio, Trifluorethylthio, Chlortrifluorethylthio, Tetrafluorethylthio, Trifluormethoxy, Difluormethoxy, Tetrafluorethoxy, Chlortrifluorethoxy und Bromtrifluorethoxy (vorzugsweise Trifluormethyl und Trifluormethoxy).

Als gegebenenfalls substituiertes Aryloxy $R^1$ und $R^2$ steht Aryloxy mit 6 bis 10 Kohlenstoffatomen im Arylteil, insbesondere 6 Kohlenstoffatomen im Arylteil. Beispielhaft seien gegebenenfalls substituiertes Phenoxy oder Naphthyloxy (vorzugsweise Phenoxy) genannt.

Die gegebenenfalls substituierten Aryloxyreste $R^1$ und $R^2$ können einen oder mehrere, vorzugsweise 1 bis 3, insbesondere 1 oder 2 gleiche oder verschiedene Substituenten tragen.
Als Arylsubstituenten seien beispielhaft aufgeführt: Halogen, vorzugsweise Fluor, Chlor, Brom oder Iod,

insbesondere Chlor und Brom; Cyano; Nitro; Alkyl mit 1 bis 4, insbesondere 1 oder 2 Kohlenstoffatomen, wie Methyl, Ethyl, n- und i-Propyl und n-, i-, sec.- und tert.-Butyl; Alkoxy mit 1 bis 4, insbesondere 1 oder 2 Kohlenstoffatomen, wie Methoxy, Ethoxy, n- und i-Propyloxy und n-, i-, sec.- und tert.-Butyloxy; Alkylthio mit 1 bis 4, insbesondere 1 oder 2 Kohlenstoffatomen, wie Methylthio, Ethylthio, n- und i-Propylthio und n-, i-, sec.- und tert.-Butylthio; Halogenalkyl, Halogenalkoxy und Halogenalkylthio mit jeweils 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Trifluormethoxy und Trifluormethylthio. Bevorzugte Substituenten sind Trifluormethyl und Chlor.

Gegebenenfalls substituiertes Alkylen in der Definition von $R^1$ und $R^2$, welches durch 1 oder 2 Sauerstoffatome unterbrochen wird oder (bevorzugt) über 1 oder 2 Sauerstoffatome an den Phenylring gebunden ist, enthält 1 bis 3, insbesondere 1 oder 2 Kohlenstoffatome und kann durch $C_1$-$C_4$-Alkyl und/oder Halogen substituiert sein. Beispielhaft seien genannt: $-O-CF_2CClF-O-$, $-O-CH_2-O-$ und $-CH_2-O-CH_2-O-$.

Als gegebenenfalls substituiertes Cycloalkyl $R^5$ steht Cycloalkyl mit 3 bis 8 Kohlenstoffatomen, insbesondere 3 bis 6 Kohlenstoffatomen im Cycloalkylring. Beispielhaft seien gegebenenfalls substituiertes Cyclopropyl, Cyclobutyl, Cyclopentyl oder Cyclohexyl genannt.

Der gegebenenfalls substituierte Cycloalkylrest $R^5$ kann einen oder mehrere, vorzugsweise 1 bis 7, insbesondere 3, 4 oder 5 gleiche oder verschiedene Substituenten tragen. Als Substituenten für $R^5$ seien beispielhaft aufgeführt: Halogen, vorzugsweise Fluor, Chlor, Brom oder Iod, insbesondere Fluor und Chlor; Alkyl mit 1 oder 2 Kohlenstoffatomen, wie Methyl und Ethyl, insbesondere Methyl.

Halogen steht, wo nicht anders erläutert, für Fluor, Chlor, Brom und Iod, vorzugsweise für Chlor und Fluor, wobei die Halogenatome jeweils gleich oder verschieden sein können.

$R^1$ und $R^2$ stehen vorzugsweise für Wasserstoff, Fluor und/oder Chlor.

Die Reste $R^1$ und $R^2$ stehen vorzugsweise in der 2-, 4-, 2,4-, 3,4- oder 2,6-Position des Phenylringes.

Die Reste $R^3$ und $R^4$ stehen vorzugsweise für Wasserstoff.

Der Rest $R^5$ steht vorzugsweise für substituiertes Cyclopropyl und Cyclobutyl, wobei als Substituenten vorzugsweise Methyl, Fluor und/oder Chlor stehen.

$R^5$ steht vorzugsweise für 2,2-Difluor-1-methyl-cyclopropyl-1-yl oder für den Rest

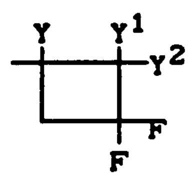

worin

| | |
|---|---|
| Y | für Wasserstoff, Methyl, Fluor oder Chlor steht und |
| $Y^1$ und $Y^2$ | gleich oder verschieden sind und für Fluor oder Chlor stehen. |

Besonders bevorzugt steht $R^5$ für 2-Chlor-2,3,3-trifluor-cyclobutyl ($Y = H$, $Y^1 = Cl$, $Y^2 = F$), 2-Chlor-1-methyl-2,3,3-trifluor-cyclobutyl ($Y = CH_3$, $Y^1 = Cl$, $Y^2 = F$) oder 2,2,3,3-Tetrafluor-cyclobutyl ($Y = H$, $Y^1$ und $Y^2 = F$).

In den allgemeinen Formeln (I), (III) und (V) befindet sich im Phenylring der Rest $R^5$ vorzugsweise in 4-Stellung zur $-NH-$, $-NH_2$ oder $-NCX$-Gruppe.

X bedeutet vorzugsweise Sauerstoff.

Die neuen Verbindungen der Formel (I) verfügen über Eigenschaften, die ihre Verwendung als Schädlingsbekämpfungsmittel ermöglichen, insbesondere zeichnen sie sich durch eine hervorragende insektizide und akarizide Wirksamkeit aus.

Besonders bevorzugt werden die neuen substituierten Furazane der Formel (I), in welcher

| | |
|---|---|
| $R^1$ und $R^2$ | gleich oder verschieden sind und für Wasserstoff, Fluor, Chlor, Brom, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy, Halogen-$C_1$-$C_4$-alkylthio oder für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy und/oder Halogen-$C_1$-$C_4$-alkylthio substituiertes Phenoxy stehen, oder |
| $R^1$ und $R^2$ | gemeinsam für einen gegebenenfalls durch Fluor, Chlor und/oder Methyl substituierten $C_1$-$C_3$-Alkylen-Rest, welcher durch 1 oder 2 Sauerstoffatome unterbrochen wird oder über 1 |

oder 2 Sauerstoffatome an den Phenylring gebunden ist, stehen,

$R^3$ und $R^4$ gleich oder verschieden sind und für Wasserstoff, Fluor, Chlor, Brom, $C_1$-$C_4$-Alkyl, $C_1$-$C_4$-Alkoxy, Halogen-$C_1$-$C_4$-alkyl oder Halogen-$C_1$-$C_4$-alkoxy stehen,

$R^5$ für gegebenenfalls durch Fluor, Chlor, Brom und/oder Methyl substituiertes Cycloalkyl mit 3 bis 6 Kohlenstoffatomen steht und

X für Sauerstoff oder Schwefel steht.

Ganz besonders bevorzugt werden die neuen substituierten Furazane der Formel (I), in welcher

$R^1$ und $R^2$ gleich oder verschieden sind und für Wasserstoff, Fluor, Chlor, Brom, Methoxy, Ethoxy, Methyl, Ethyl, n-Propyl, i-Propyl, n-Butyl, i-Butyl, sec.-Butyl, tert.-Butyl, Methylthio, Ethylthio, Trifluormethyl, Trifluormethoxy, Difluormethyl, Difluorethyl, Pentafluorethyl, Difluormethoxy, Chlordifluormethoxy, Chlortrifluorethoxy, Tetrafluorethoxy, Dichlordifluorethoxy, Difluortrichlorethoxy, Hexafluorpropoxy, Trifluormethylthio, Difluormethylthio, Chlordifluormethylthio, Chlortrifluorethylthio, Hexafluorpropylthio oder für gegebenenfalls durch Fluor, Chlor, Brom, Nitro, Cyano, Methyl, Ethyl, n-Propyl, i-Propyl, n-Butyl, i-Butyl, sec.-Butyl, tert.-Butyl, Methoxy, Ethoxy, n-Propoxy, i-Propoxy, n-Butoxy, i-Butoxy, sec.-Butoxy, tert.-Butoxy, Methylthio, Ethylthio, Trifluormethyl, Trifluormethoxy, Difluormethyl, Difluorethyl, Pentafluorethyl, Difluormethoxy, Chlordifluormethoxy, Chlortrifluorethoxy, Tetrafluorethoxy, Dichlordifluorethoxy, Difluortrichlorethoxy, Hexafluorpropoxy, Trifluormethylthio, Difluormethylthio, Chlordifluormethylthio, Chlortrifluorethylthio und/oder Hexafluorpropylthio substituiertes Phenoxy stehen, oder

$R^1$ und $R^2$ gemeinsam für Difluormethylendioxy, Ethylendioxy, Chlortrifluorethylendioxy, Difluorethylendioxy, Methylendioxy, Trifluorethylendioxy, Difluormethylenoxydifluormethylenoxy, Tetrafluorethylendioxy, 2-Methyl-1,1,2-trifluorethylendioxy oder 2,2-Dimethylethylenoxy stehen,

$R^3$ und $R^4$ gleich oder verschieden sind und für Wasserstoff, Fluor, Chlor, Brom, Methyl, Ethyl, n-Propyl, i-Propyl, n-Butyl, i-Butyl, sec.-Butyl, tert.-Butyl, Methoxy, Ethoxy, Trifluormethyl, Trifluormethoxy, Difluormethyl, Difluorethyl, Pentafluorethyl, Difluormethoxy, Chlordifluormethoxy, Chlortrifluorethoxy, Tetrafluorethoxy, Dichlordifluorethoxy, Difluortrichlorethoxy oder Hexafluorpropoxy stehen,

$R^5$ für gegebenenfalls durch Fluor, Chlor, Brom und/oder Methyl substituiertes Cyclopropyl, Cyclobutyl, Cyclopentyl oder Cyclohexyl steht und

X für Sauerstoff oder Schwefel steht.

Von besonderem Interesse sind diejenigen Verbindungen der Formel (I), in welcher

$R^1$ und $R^2$ gleich oder verschieden sind und für Wasserstoff, Fluor, Chlor, Brom, Methyl, Methoxy, Methylthio, Trifluormethyl, Trifluormethoxy, Trifluormethylthio oder für gegebenenfalls durch Chlor, Methyl, Methoxy, Methylthio, Trifluormethyl, Trifluormethoxy und/oder Trifluormethylthio substituiertes Phenoxy stehen, oder

$R^1$ und $R^2$ gemeinsam für Methylendioxy, Ethylendioxy, Difluormethylendioxy, Tetrafluorethylendioxy oder Chlortrifluorethylendioxy stehen,

$R^3$ und $R^4$ gleich sind und für Wasserstoff stehen,

$R^5$ für durch Fluor, Chlor und/oder Methyl substituiertes Cyclopropyl und Cyclobutyl steht und

X für Sauerstoff oder Schwefel steht.

Von ganz besonderem Interesse sind die Verbindungen der Formel (I), in welcher

$R^1$ und $R^2$ gleich oder verschieden sind und für Wasserstoff, Fluor oder Chlor stehen,

$R^2$ und $R^3$ für Wasserstoff stehen,

$R^5$ für 2-Chlor-2,3,3-trifluor-cyclobutyl, 1-Methyl-2-chlor-2,3,3-trifluorcyclobutyl oder 2,2,3,3-Tetrafluor-cyclobutyl stehen und

X für Sauerstoff oder Schwefel (vorzugsweise Sauerstoff) stehen.

Verwendet man nach Verfahrensvariante (a) 3-Amino-4-(4-chlorphenyl)-1,2,5-oxadiazol und 4-(2,2,3,3-Tetrafluorcyclobut-1-yl)-phenylisocyanat als Ausgangsstoffe, so kann der Reaktionsablauf durch das folgende Formelschema wiedergegeben werden:

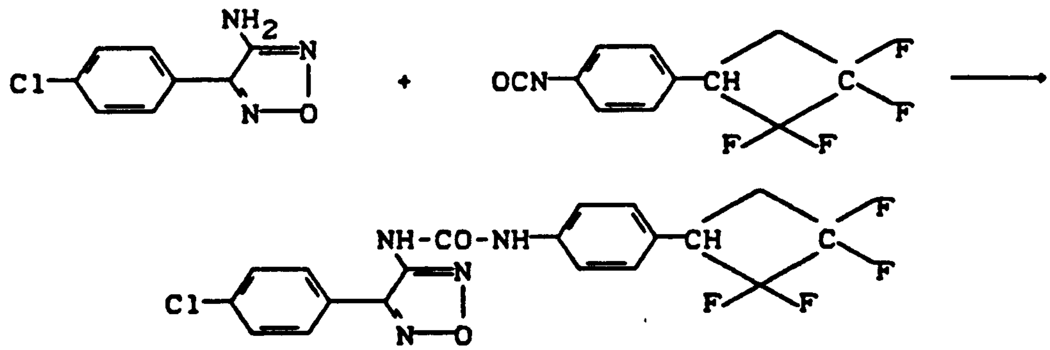

Verwendet man nach Verfahrensvariante (b) 4-(4-Chlorphenyl)-3-isocyanato-1,2,5-oxadiazol und 4-(2-Chlor-1-methyl-2,3,3-trifluor-cyclobut-1-yl)-anilin als Ausgangsstoffe, so kann der Reaktionsablauf durch das folgende Formelschema wiedergegeben werden:

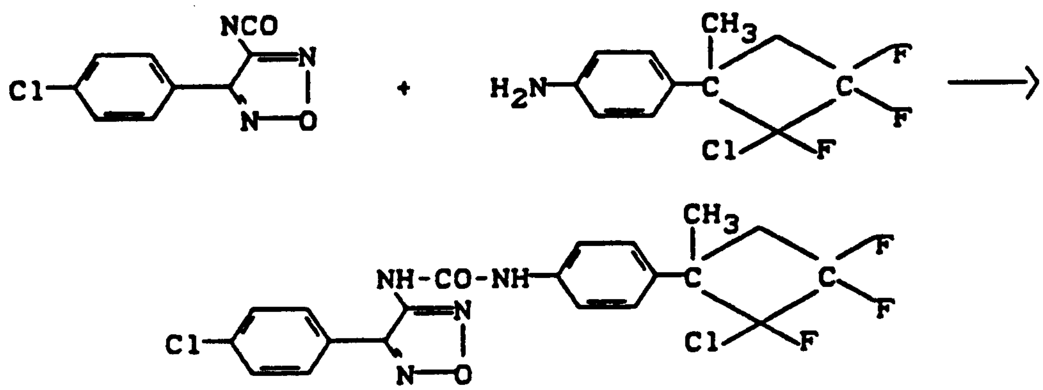

Die als Ausgangsstoffe zu verwendenden 3-Amino-1,2,5-oxadiazole der Formel (II) sind bekannt und/oder lassen sich nach literaturbekannten Verfahren und Methoden herstellen (vergl. J. Prakt. Chem. 315, 4, Seite 791 - 795 (1973)). Die Aminogruppe kann nach üblichen Verfahren in die Isocyanat- bzw. Isothio-cyanat-Gruppe umgewandelt werden, z. B. durch Umsetzung mit Phosgen bzw. Thiophosgen in Verdünnungsmitteln wie z. B. Toluol und/oder Pyridin, bei einer Temperatur zwischen -20 °C und +50 °C. Man erhält auf diese Weise die Verbindungen der Formel (IV).

Die Verbindungen der Formeln (III) und (V) sind zum Teil bekannt (vergl. z. B. J. Am. Chem. Soc. 93, 7208 ff. (1971) und Zh. Org. Khim. 10, 477 - 483 (1974)). Die neuen Verbindungen der Formeln (III) und (V) werden weiter unten beschrieben.

Als Verdünnungsmittel kommen beim erfindungsgemäßen Verfahren (Varianten (a) und (b)) praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Tetramethylensulfon.

Als Katalysatoren können für die Umsetzung gemäß Verfahrensvarianten (a) und (b) vorzugsweise tertiäre Amine, wie Triethylamin und 1,4-Diazabicyclo-[2,2,2]-octan, sowie organische Zinn-Verbindungen, wie z. B. Dibutylzinndilaurat verwendet werden. Im allgemeinen bringt der Zusatz des Katalysators keine Vorteile.

Die Reaktionstemperatur kann innerhalb eines größeren Bereiches variiert werden. Bei der Verfahrensvariante (a) arbeitet man zwischen 20 °C und 180 °C, vorzugsweise zwischen 40 °C und 120 °C und bei der Verfahrensvariante (b) zwischen 20 °C und 200 °C, vorzugsweise zwischen 60 °C und 190 °C. Die erfindungsgemäßen Verfahrensvarianten werden im allgemeinen bei Normaldruck durchgeführt.

Zur Durchführung der erfindungsgemäßen Verfahrensvarianten werden werden die Ausgangsstoffe

gewöhnlich in etwa äquimolaren Mengen eingesetzt. Ein Überschuß der einen oder anderen Reaktionskomponente bringt keine wesentlichen Vorteile. Vorzugsweise setzt man auf 1 Mol der Verbindungen der Formel (II) bzw. (IV) 0,6 bis 1,5 Mol, insbesondere 0,8 bis 1,2 Mol der Verbindung der Formel (III) bzw. (V) ein.

Die Aufarbeitung der Reaktionsprodukte geschieht nach üblichen Methoden, z. B. durch Absaugen des ausgefallenen Produktes oder durch Herauslösung von unerwünschten Nebenprodukten aus dem Reaktionsgemisch. Zur Charakterisierung dient der Schmelzpunkt.

Die Verbindungen der Formeln (III) und (V) sind zum Teil neu. Die neuen Verbindungen können unter der allgemeinen Formel (VI) zusammengefaßt werden:

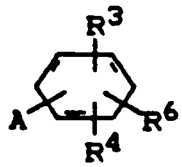

(VI)

in welcher

A für $-NH_2$ oder -NCX steht, wobei

X für Sauerstoff oder Schwefel steht,

$R^3$ und $R^4$ gleich oder verschieden sind und für Wasserstoff, Halogen, Alkyl, Alkoxy, Halogenalkyl oder Halogenalkoxy stehen und

$R^6$ für 2,2-Difluor-1-methyl-cycloprop-1-yl oder für den Rest

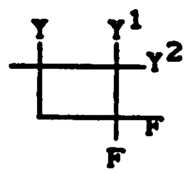

steht, worin

Y für Wasserstoff, Methyl, Fluor oder Chlor steht und

$Y^1$ und $Y^2$ gleich oder verschieden sind und für Fluor oder Chlor stehen.

Diese Verbindungen der Formel (VI) sowie die Verfahren zu ihrer Herstellung sind Teil der vorliegenden Erfindung.

Die Verbindungen der allgemeinen Formel (VI) werden dadurch erhalten, daß man Nitrobenzole der allgemeinen Formel (VII)

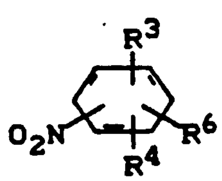

(VII)

in welcher

$R^3$ und $R^4$ gleich oder verschieden sind und für Wasserstoff, Halogen, Alkyl, Alkoxy, Halogenalkyl oder Halogenalkoxy stehen und

$R^6$ für 2,2-Difluor-1-methyl-cycloprop-1-yl oder für den Rest

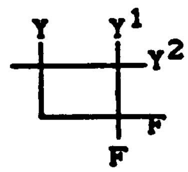

steht, worin

Y für Wasserstoff, Methyl, Fluor oder Chlor steht und

$Y^1$ und $Y^2$ gleich oder verschieden sind und für Fluor oder Chlor stehen,

in üblicher Weise reduziert, vorzugsweise durch katalytische Hydrierung oder mit Hilfe von Metallen oder Metallsalzen und gegebenenfalls die entstandene Aminoverbindung mit Phosgen oder Thiophosgen umsetzt.

Für die katalytische Hydrierung der Nitroverbindungen der allgemeinen Formel (VII) kommen alle üblichen Katalysatoren in Frage, wie Raney-Nickel, Platin, Platinoxid und Palladium, wobei die Katalysatoren auch auf Trägern, z. B. Aktivkohle oder Aluminiumoxid vorliegen können.

Als Lösungsmittel kommen alle bei Hydrierungen üblicherweise eingesetzten Lösungsmittel in Frage, wie Alkohole, z. B. Methanol oder Ether, z. B. Dioxan und Tetrahydrofuran.

Im allgemeinen setzt man erhöhte Wasserstoffdrücke, vorzugsweise zwischen 10 und 80 bar ein. Die Hydrierung wird vorzugsweise bei Temperaturen von 10 °C bis 100 °C, insbesondere 20 °C bis 80 °C durchgeführt. Die Aufarbeitung und Isolierung der Verbindungen der allgemeinen Formel (VI) (A bedeutet $-NH_2$) bzw. (V) erfolgt in üblicher Weise.

Die Reduktion der Nitroverbindungen der allgemeinen Formel (VII) kann in üblicher Weise auch mit Metallen wie Eisen und Zinn und deren Salzen, vorzugsweise $SnCl_2$, vorgenommen werden. Die Reduktion mit $SnCl_2$ erfolgt vorzugsweise in einem Gemisch aus wäßrigen HC1 und Dioxan (oder auch wasserfrei in Alkoholen, wie Ethanol) bei Temperaturen zwischen 20 °C und 100 °C. Analog kann auch Eisen in Form von Eisenpulver oder von Eisenspänen verwendet werden, wobei vorzugsweise Temperaturen von 80 °C bis 100 °C eingesetzt werden. Die Aufarbeitung und Isolierung der Verbindungen der allgemeinen Formel (VI) (A bedeutet $-NH_2$) bzw. (V) erfolgt auch hierbei in üblicher Weise.

Die Verbindungen der allgemeinen Formel (VI) (A bedeutet -NCX) bzw. (III) können nach den üblichen Phosgenierungsmethoden leicht aus den Verbindugen der allgemeinen Formel (VI) (A bedeutet $-NH_2$) bzw. (V) und Phosgen bzw. Thiophosgen erhalten werden. Als Lösungsmittel werden vorzugsweise gegebenenfalls halogenierte aromatische oder aliphatische Lösungsmittel wie Chlortoluol, Toluol oder Xylol verwendet.

Die Phosgenierung erfolgt vorzugsweise bei Temperaturen zwischen 0 °C und 150 °C, insbesondere zwischen 0 °C und 130 °C.

Die Aufarbeitung und Isolierung der Verbindungen der allgemeinen Formel (VI) (A bedeutet -NCX) bzw. (V) wird nach allgemein üblichen Methoden vorgenommen.

Die Verbindungen der allgemeinen Formel (VII) sind neu. Diese Verbindungen sowie das Verfahren zur ihrer Herstellung sind ebenfalls Teil der vorliegenden Erfindung.

Die Verbindungen der allgemeinen Formel (VII) werden dadurch erhalten, daß man Benzole der Formel (VIII)

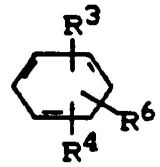

(VIII)

in welcher

$R^3$ und $R^4$ gleich oder verschieden sind und für Wasserstoff, Halogen, Alkyl, Alkoxy, Halogenalkyl oder Halogenalkoxy stehen und

$R^6$ für 2,2-Difluor-1-methyl-cycloprop-1-yl oder für den Rest

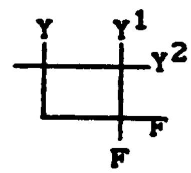

steht, worin

Y für Wasserstoff, Methyl, Fluor oder Chlor steht und

$Y^1$ und $Y^2$ gleich oder verschieden sind und für Fluor oder Chlor stehen,

nach allgemein üblichen Methoden der Kernnitrierung nitriert. Vorzugsweise wird bei Temperaturen zwischen 0°C und 50°C, insbesondere zwischen 5°C und 40°C die Nitriersäure (Mischung aus konz. Schwefelsäure und konz. Salpetersäure) ohne die Verwendung eines Lösungsmittels langsam zu der Verbindung der allgemeinen Formel (VIII) hinzugefügt und das Reaktionsgemisch 1 bis 3 Stunden bei dieser Temperatur gehalten und gegebenenfalls gegen Reaktionsende mit einem Lösungsmittel, wie z. B. Dichlormethan versetzt. Anschließend wird das Reaktionsgemisch auf Eis gegossen, wobei sich das gewünschte Produkt als organische Phase abscheidet und leicht abtrennen läßt. Die Reinigung der Verbindungen der Formel (VII) erfolgt nach üblichen Methoden.

Die Verbindungen der Formel (VIII) sind bekannt und/oder lassen sich nach bekannten Verfahren herstellen (vergl. z. B. J. Am. Chem. Soc. 83, 382 (1961) und 86, 2645 (1964)).

Die neuen Verbindungen der allgemeinen Formeln (VI) und (VII) können in der folgenden allgemeinen Formel (IX) zusammengefaßt werden:

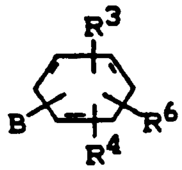

(IX)

in welcher

B für $-NH_2$, $-NO_2$ oder-NCX steht, worin

X für Sauerstoff oder Schwefel steht.

$R^3$ und $R^4$ gleich oder verschieden sind und für Wasserstoff, Halogen, Alkyl, Alkoxy, Halogenalkyl oder Halogenalkoxy stehen und

$R^6$ für 2,2-Difluor-1-methyl-cycloprop-1-yl oder für den Rest

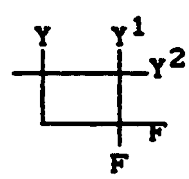

steht, worin

Y für Wasserstoff, Methyl, Fluor oder Chlor steht und

$Y^1$ und $Y^2$ gleich oder verschieden sind und für Fluor oder Chlor stehen.

Die bevorzugten Definitionen und Stellungen der Reste in den Verbindungen der allgemeinen Formeln (II) bis (V) und bezüglich der Reste $R^1$, $R^2$, $R^3$, $R^4$ und X auch in den Verbindungen der allgemeinen Formeln (VI) bis (IX) entsprechen denen, welche für die Verbindungen der allgemeinen Formel (I) weiter oben angegeben wurden.

Die Wirkstoffe eignen sich zur Bekämpfung von tierischen Schädlingen, vorzugsweise Arthropoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen

gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe der Formel (I) zeichenen sich durch eine hervorragende insektizide und akarizide Wirksamkeit, vorzugsweise gegen Eier (ovizide Wirkung) und die Larven (larvizide Wirkung) der Schädlinge, aus. Sie zeigen insbesondere beim Einsatz als Akarizide eine hervorragende entwicklungshemmende Wirkung auf Eier und/oder Larven von Tetranychiden, wie z.B. Tetranychus urticae. Eine sehr gute Wirkung zeigen die erfindungsgemäßen Wirkstoffe auch bei der Bekämpfung von pflanzenschädigenden Insekten, wie z. B. Phaedon cochleariae-Larven.

Die Wirkstoffe können in die üblichen Formulierungen übergeführt werden, wie Lösungen, Emulsionen,

Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Herstellung der erfindungsgemäßen Verbindungen sowie die biologische Wirksamkeit der erfindungsgemäßen Verbindungen der Formel (I) soll anhand der folgenden Beispiele erläutert werden.

Herstellungsbeispiele

Beispiel 1

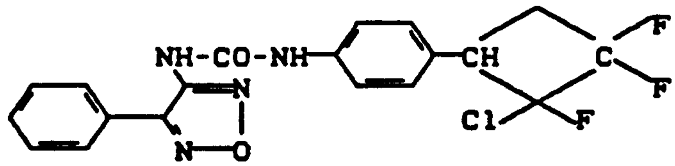

(Verfahrensvariante (b))

Zu einer Lösung von 2,36 g (0,01 Mol) 4-(2-Chlor-2,3,3-trifluor-cyclobutyl)-anilin in 40 ml Toluol fügt man unter Feuchtigkeitsausschluß bei 40 °C 1,87 g (0,01 Mol) 3-Isocyanato-4-phenyl-1,2,5-oxadiazol gelöst in 10 ml trockenem Toluol.

Der Ansatz wird eine halbe Stunde bei 80 °C gerührt und anschließend im Vakuum eingeengt. Das ausgefallene Produkt wird abgesaugt und mit Petrolether gewaschen.

Man erhält 3,2 g (76 % der Theorie) 1-[4-(2-Chlor-2,3,3-trifluor-cyclobutyl)-phenyl]-3-(4-phenyl-1,2,5-oxadiazol3-yl)-harnstoff vom Schmelzpunkt 201 °C.

Analog Beispiel 1 bzw. Verfahrensvariante (a) oder (b) werden die in der nachfolgenden Tabelle 1 aufgeführten Verbindungen der Formel (I) hergestellt:

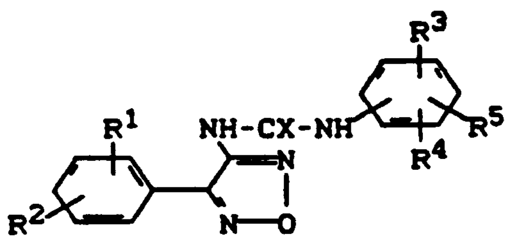

(I)

**Tabelle 1**

| Beisp.-Nr. | $R^1$ | $R^2$ | $R^3$ | $R^4$ | $R^5$ | X | Schmelzpunkt /[°C] |
|---|---|---|---|---|---|---|---|
| 2 | 4-Cl | H | H | H | 4- 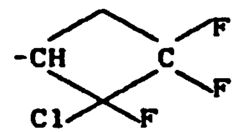 | O | 216 |
| 3 | 4- 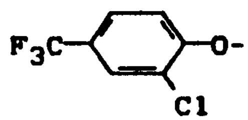 | H | H | H | 4- 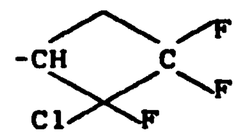 | O | 114 |
| 4 | H | H | H | H | 4- 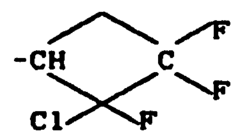 | S | 112 |
| 5 | 4-F | H | H | H | 4- 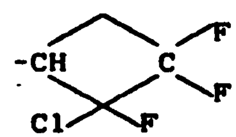 | O | 226 |
| 6 | 4-Br | H | H | H | 4- 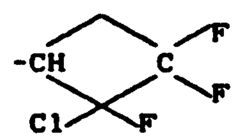 | O | 221 |
| 7 | 2-F | 4-F | H | H | 4- 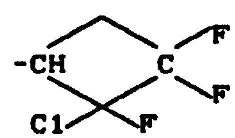 | O | 186 |

Tabelle 1 - Fortsetzung

| Beisp.-Nr. | $R^1$ | $R^2$ | $R^3$ | $R^4$ | $R^5$ | X | Schmelzpunkt /[°C] |
|---|---|---|---|---|---|---|---|
| 8 | 2-Cl | 4-$CF_3$ | H | H | 4- 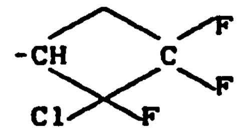 | O | 203 |
| 9 | H | H | H | H | 4- 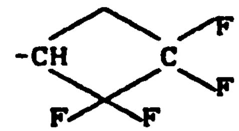 | O | 206 - 208 |
| 10 | 4-F | H | H | H | 4- 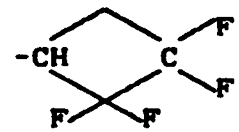 | O | 225 |
| 11 | 4-Br | H | H | H | 4- 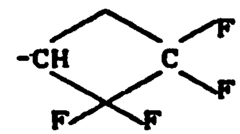 | O | 248 |
| 12 | 4-Cl | H | H | H | 4- 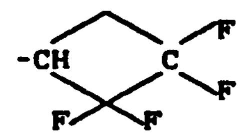 | O | 232 |
| 13 | 4-$CH_3$ | H | H | H | 4- 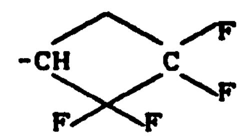 | O | 223 |

<u>Tabelle 1</u> - Fortsetzung

| Beisp.-Nr. | $R^1$ | $R^2$ | $R^3$ | $R^4$ | $R^5$ | X | Schmelzpunkt /[°C] |
|---|---|---|---|---|---|---|---|
| 14 | 3-Cl | 4-Cl | H | H | 4- 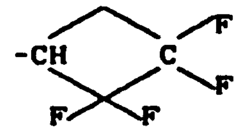 | O | 219 |
| 15 | 3,4-$OCH_2O$- | | H | H | 4- 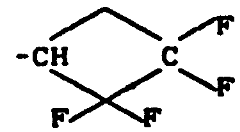 | O | 195 |
| 16 | 4-$CH_3O$- | H | H | H | 4- 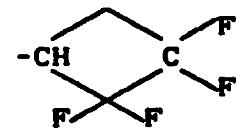 | O | 231 |
| 17 | 4- 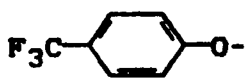 | H | H | H | 4- 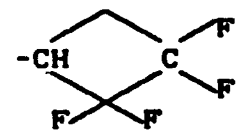 | O | 189 |
| 18 | 4- 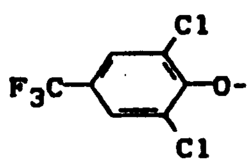 | H | H | H | 4- 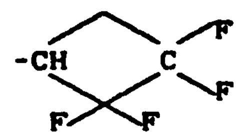 | O | 203 |
| 19 | 4- 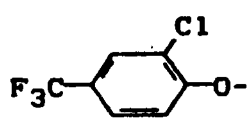 | H | H | H | 4- 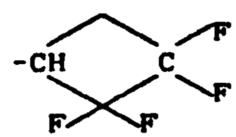 | O | 109 |

Tabelle 1 - Fortsetzung

| Beisp.-Nr. | $R^1$ | $R^2$ | $R^3$ | $R^4$ | $R^5$ | X | Schmelzpunkt /[°C] |
|---|---|---|---|---|---|---|---|
| 20 | H | H | H | H | 4- 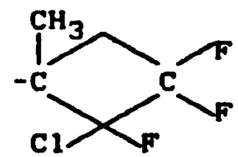 | O | 202 |
| 21 | 4-F | H | H | H | 4- 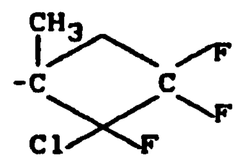 | O | 210 |
| 22 | 4-Br | H | H | H | 4- 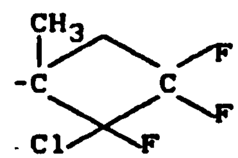 | O | 219 |
| 23 | H | H | H | H | 4- 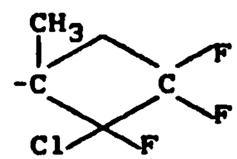 | S | 146 |
| 24 | 4-Cl | H | H | H | 4- 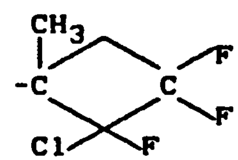 | O | 222 |

**Tabelle 1** - Fortsetzung

| Beisp.-Nr. | $R^1$ | $R^2$ | $R^3$ | $R^4$ | $R^5$ | X | Schmelzpunkt /[°C] |
|---|---|---|---|---|---|---|---|
| 25 | 2-Cl | H | H | H | 4- 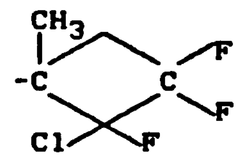 | O | 222 |
| 26 | 2-Cl | 4-Cl | H | H | 4- 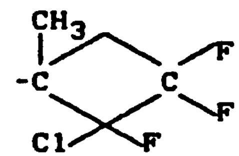 | O | 181 |
| 27 | 4-$CF_3$ | H | H | H | 4- 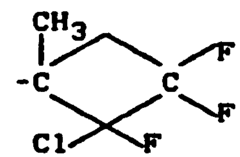 | O | 223 |
| 28 | 4-$CF_3O$- | H | H | H | 4- 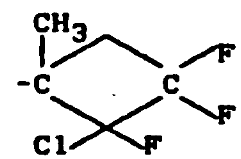 | O | 229 |
| 29 | 4-$CH_3$ | H | H | H | 4- 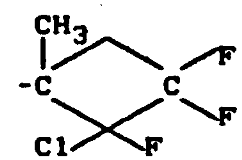 | O | 199 |

**Tabelle 1** - Fortsetzung

| Beisp.-Nr. | $R^1$ | $R^2$ | $R^3$ | $R^4$ | $R^5$ | X | Schmelzpunkt /[°C] |
|---|---|---|---|---|---|---|---|
| 30 | 4-$CH_3O$- | H | H | H | 4- 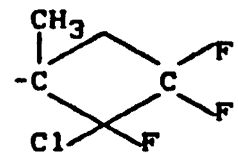 | O | 226 |
| 31 | 4- 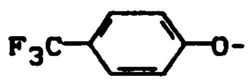 | H | H | H | 4- 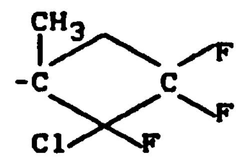 | O | 102 |
| 32 | 4-$CF_3O$- | H | H | H | 4- 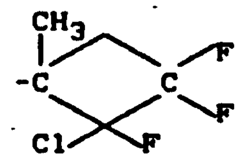 | O | 228 |
| 33 | 4-Cl | 3-Cl | H | H | 4- 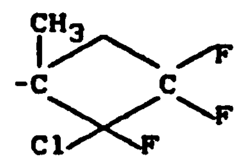 | O | 229 |
| 34 | 3,4-$OCH_2O$- | | H | H | 4- 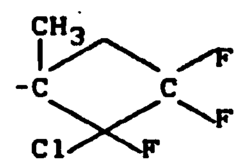 | O | 180 |

**Tabelle 1** - Fortsetzung

| Beisp.-Nr. | $R^1$ | $R^2$ | $R^3$ | $R^4$ | $R^5$ | X | Schmelzpunkt /[°C] |
|---|---|---|---|---|---|---|---|
| 35 | 4-Cl | H | H | H | 4- 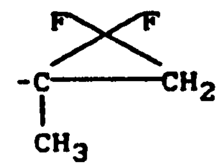 | O | 215 |
| 36 | 4-F | H | H | H | 4- 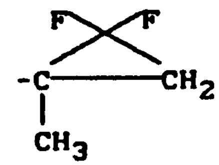 | O | 210 |
| 37 | 4-Br | H | H | H | 4- 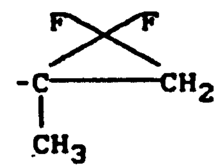 | O | 211 |
| 38 | $4-CH_3$ | H | H | H | 4- 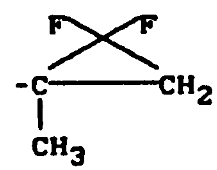 | O | 184 |
| 39 | 4-Cl | 3-Cl | H | H | 4- 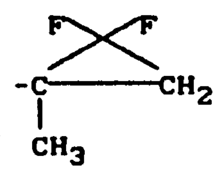 | O | 220 |

**Tabelle 1** - Fortsetzung

| Beisp.-Nr. | $R^1$ | $R^2$ | $R^3$ | $R^4$ | $R^5$ | X | Schmelzpunkt /[°C] |
|---|---|---|---|---|---|---|---|
| 40 | 3,4-O-$CH_2$-O- | | H | H | 4- 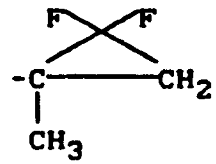 | O | 177 |
| 41 | 4- 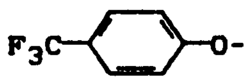 | H | H | H | 4- 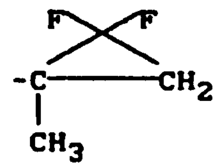 | O | 101 |
| 42 | H | H | H | H | 2- 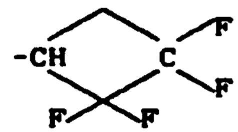 | O | 184 |
| 43 | 4-F | H | H | H | 2- 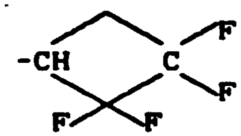 | O | 195 |
| 44 | H | H | H | 3-$CF_3$ | 4- 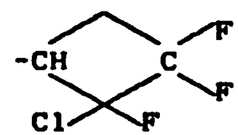 | O | 210 |
| 45 | 4-Cl | H | H | 3-$CF_3$ | 4- 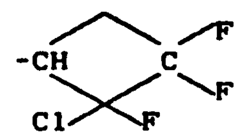 | O | 219 |

**Tabelle 1** - Fortsetzung

| Beisp.-Nr. | $R^1$ | $R^2$ | $R^3$ | $R^4$ | $R^5$ | X | Schmelzpunkt /[°C] |
|---|---|---|---|---|---|---|---|
| 46 | 4-F | H | H | $3-CF_3$ | 4- 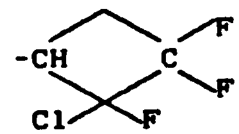 | O | 207 |
| 47 | 4- 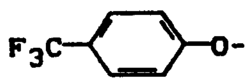 | H | H | $3-CF_3$ | 4- 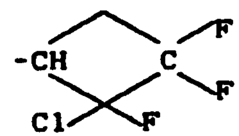 | O | 145 |
| 48 | $4-CH_3O-$ | H | H | $3-CF_3$ | 4- 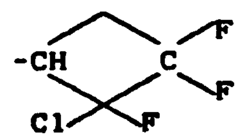 | O | 206 |
| 49 | $3,4-O-CH_2-O-$ | | H | $3-CF_3$ | 4- 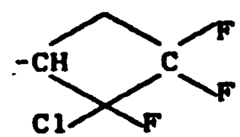 | O | 228 |

Herstellungsbeispiel gemäß Verfahrensvariante (a) für die Verbindung aus Beispiel 9

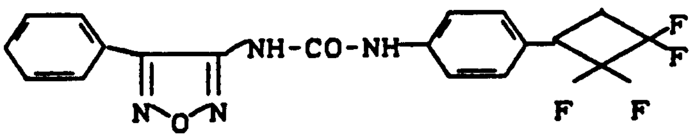

3,22 g (0,02 Mol) 3-Amino-4-phenyl-1,2,5-oxadiazol werden in 10 ml trockenem Dimethylformamid gelöst. Zu dieser Lösung fügt man 5,4 g (0,022 Mol) 4-(2,2,3,3-Tetrafluorcyclobut-1-yl)-phenylisocyanat. Der Ansatz wird 6 Stunden bei 100 ° C gerührt. Nach dem Abkühlen wird das Produkt durch Zutropfen von Wasser ausgefällt. Es wird mehrfach mit Wasser gewaschen. Das von der wäßrigen Phase abgetrennte Produkt wird mit Toluol:Petrolether = 1:3 abgedeckt und bei 80 ° C getrocknet.

Man erhält 7,9 g (94,5 % der Theorie) 1-[4-(2,2,3,3,-Tetrafluorcyclobutyl)-phenyl]-3-(4-phenyl-1,2,5-oxadiazol-3-yl)-harnstoff mit einem Schmelzpunkt von 206 ° C.

Ausgangsstoffe der Formel (VI) (A = $-NH_2$)

Beispiel (VI-1)

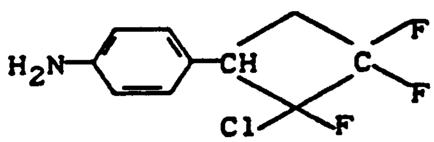

In einem Hydrierautoklaven werden 158,0 g 4-(2-Chlor-2,3,3-trifluorcyclobutyl)-nitrobenzol in 400 ml Methanol vorgelegt, 10 g Raney-Nickel zugegeben und bei 25 ° C - 40 ° C mit einem Wasserstoffdruck von 30 bar hydriert. Anschließend wird der Katalysator abfiltriert, das Lösungsmittel im Vakuum entfernt und der Rückstand destilliert.

Man erhält 128 g 4-(2-Chlor-2,3,3-trifluorcyclobut-1-yl)-anilin vom Siedepunkt Kp: 157 - 160 ° C / 20 mbar (2000 Pa).

Analog Beispiel (VI-1) können die folgenden Verbindungen der Formel (VI) (A = $-NH_2$) erhalten werden:

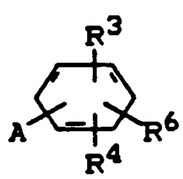

(VI)

Beispiel (VI-2)

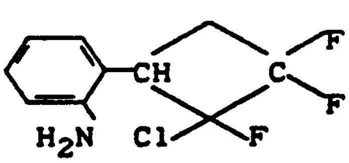

Kp: 97 - 99 °C/0,1 mbar (10 Pa)

$n_D^{20}$: 1,5285

Beispiel (VI-3)

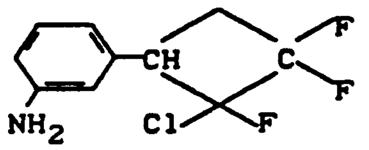

Kp: 101 - 102 °C/0,1 mbar (10 Pa)

$n_D^{20}$: 1,5250

Beispiel (VI-4)

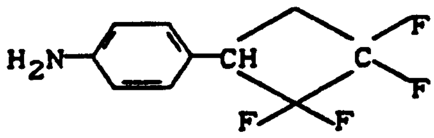

Fp: 52 - 53 °C

Beispiel (VI-5)

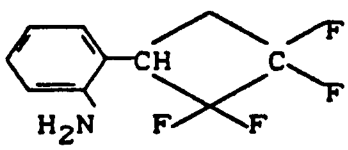

Fp: 64 - 65 °C

Beispiel (VI-6)

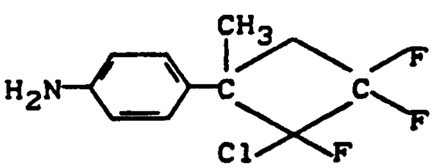

Kp: 154 °C/14 mbar (1400 Pa)

$n_D^{20}$: 1,5235

Beispiel (VI-7)

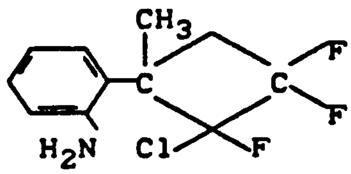

Kp: 150 - 151 °C/20 mbar (2000 Pa)

$n_D^{20}$: 1,5268

Beispiel (VI-8)

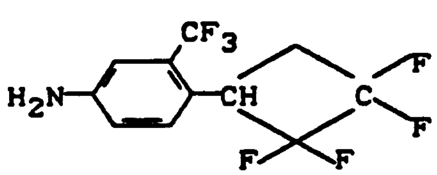

Kp: 122 - 124 °C/2 mbar (200 Pa)

Beispiel (VI-9)

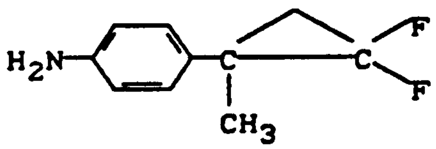

Kp: 118 - 120 °C/14 mbar (1400 Pa)

$n_D^{20}$: 1,5223

Ausgangsstoffe der Formel (VI) (A = -NCX)

Beispiel (VI-10)

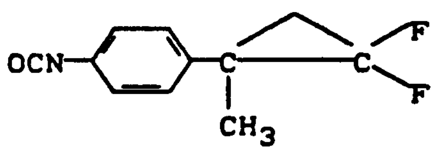

In 500 ml Chlorbenzol werden 42 g 4-(2,2-Difluor-1-methylcycloprop-1-yl)-anilin bei ca. 5 °C vorgelegt und 40 g Phosgen eingeleitet, wobei die Temperatur erhöht wird. Ist der Siedepunkt der Lösung erreicht, wird mit Stickstoff ausgeblasen. Das Reaktionsgemisch wird eingeengt und das Rohprodukt durch Destillation gereinigt.

Man erhält 41 g 4-(2,2-Difluor-1-methyl-cycloprop-1-yl)-phenylisocyanat vom Siedepunkt Kp: 120 °C/16 mbar (1600 Pa).

Beispiel (VI-11)

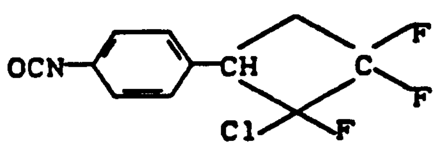

$n_D^{20}$: 1,5120

Beispiel (VI-12)

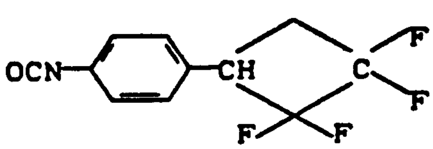

Kp: 96 °C/2 mbar (200 Pa)

$n_D^{20}$: 1,4879

Ausgangsprodukte der Formel (VII)

Beispiel (VII-1)

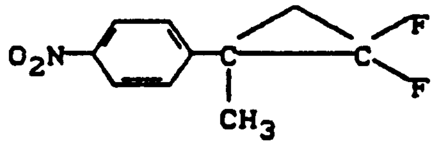

In einer Nitrierapparatur mit Rührer und Tropftrichter werden 80 g 2,2-Difluor-1-methyl-1-phenyl-cyclopropan (Herstellung siehe unten) bei 5 °C vorgelegt und eine Mischung aus 50 g 67 %iger Salpetersäure und 60 g konz. Schwefelsäure zugetropft. Anschließend wird eine Stunde bei 10 °C und 1 Stunde bei 20 °C nachgerührt und auf Eis gegossen. Die organische Phase wird abgetrennt, getrocknet und eingeengt.

Man erhält 86 g rohes 4-(2,2-Difluor-1-methyl-cycloprop-1-yl)-nitrobenzol.

Analog Beispiel (VII-1) können die folgenden Verbindungen der Formel (VII) hergestellt werden:

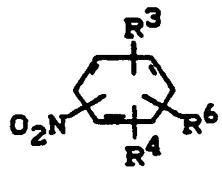

(VII)

Beispiel (VII-2)

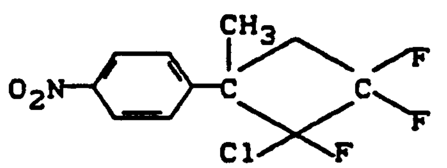

Kp: 140 - 146 °C/1,5 mbar (150 Pa)

$n_D^{20}$: 1,5195

Beispiel (VII-3)

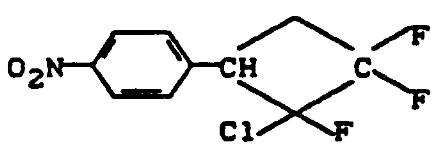

Kp: 135 - 138 °C/0,2 mbar (20 Pa)

$n_D^{20}$: 1,5200

Beispiel (VII-4)

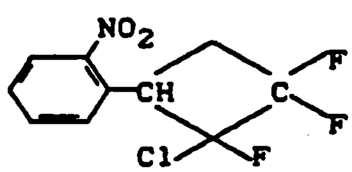

Kp: 131 - 132 °C/0,15 mbar (15 Pa)

$n_D^{20}$: 1,5230

Beispiel (VII-5)

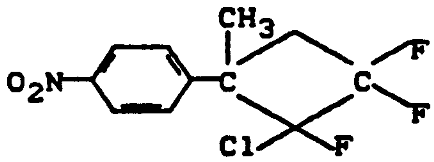

Kp: 135 - 137 °C/1 mbar (100 Pa)

$n_D^{20}$: 1,590

Beispiel (VII-6)

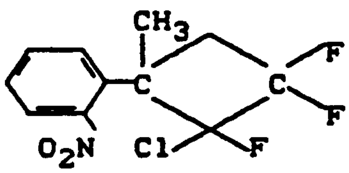

Kp: 119 - 120 °C/0,2 mbar (20 Pa)

$n_D^{20}$: 1,5215

Beispiel (VII-7)

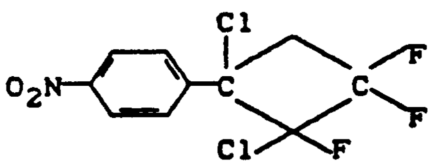

Kp: 128 - 130 °C/0,8 mbar (20 Pa)

$n_D^{20}$: 1,5480

Beispiel (VII-8)

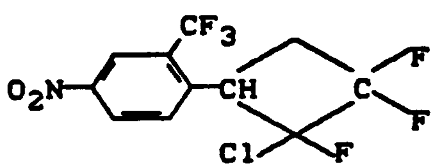

Kp: 114 - 118 °C/1,4 mbar (140 Pa)

$n_D^{20}$: 1,4865

Herstellung von 2,2-Difluor-1-methyl-1-phenyl-cyclopropan

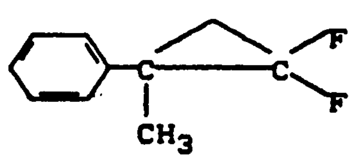

In einem Autoklaven werden 200 g 1-Methyl-styrol, 20 g Tetrabutylammoniumchlorid und 10 g Hydrochinon vorgelegt und dann 500 g Difluorchlormethan aufgedruckt, auf 130 °C erhitzt und innerhalb von einer Stunde mit 600 g Ethylenoxid versetzt. Nach 12 Stunden wird abgekühlt und das Reaktionsgemisch über eine Kolonne destilliert. Die Fraktion bei 53 °C - 65 °C/14 mbar wird dreimal mit Wasser gewaschen, getrocknet und erneut destilliert.

Man erhält so 215 g 2,2-Difluor-1-methyl-1-phenyl-cyclopropan vom Siedepunkt Kp: 85 °C/14 mbar (1400 Pa).

Die übrigen Ausgangsstoffe der allgemeinen Formel (VIII) können analog erhalten werden.

Verwendungsbeispiele

In den folgenden Verwendungsbeispielen wurden die folgenden Verbindungen als Vergleichssubstanzen eingesetzt:

(A)

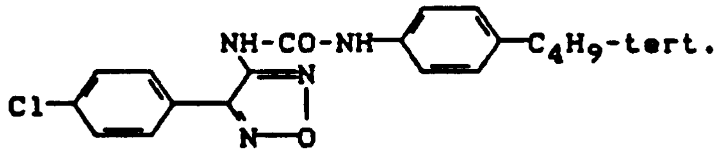

(B)

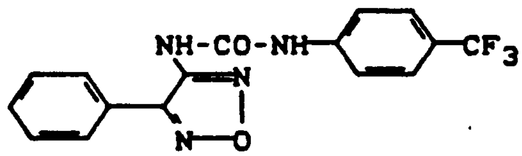

(C)

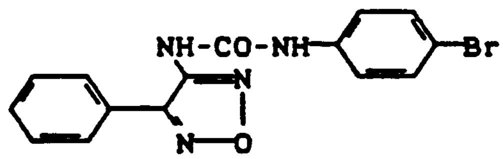

aus EP-A 00 132 680

Beispiel A

Eisterilitäts- und Entwicklungshemmtest mit Tetranychus urticae (Gemeine Spinnmilbe)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Die Blätter einer Bohnenpflanze (Phaseolus vulgaris) taucht man in die entsprechend konzentrierte Wirkstoffzubereitung. Nach Antrocknen der Wirkstoffzubereitung besetzt man die Blätter für ca. 16 Stunden mit Spinnmilben-Weibchen zur Eiablage (ca. 50 Eier/Wiederholung). Die Summe der sterilen und/oder abgetöteten Eier und der abgetöteten Larven, Nymphen und Ruhestadien einer Generation bezogen auf die Zahl der abgelegten Eier ergibt die Abtötung in %. Dabei bedeutet 100 %, daß alle Tiere abgetötet wurden; 0 % bedeutet, daß keine Tiere abgetötet wurden.

Bei diesem Test zeigten bei einer beispielhaften Wirkstoffkonzentration von 0,01 % nach 14 Tagen die Verbindungen der Herstellungsbeispiele (1), (2), (4), (5), (7), (9), (20), (21), (23) und (24) eine Wirkung von 100 %, während die Vergleichsverbindungen (A), (B) und (C) eine Wirkung von 62%, 8 % bzw. 0 % Wirkung aufwiesen.

Beispiel B

Phaedon-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlpflanzen (Brassica oleracea) werden mit der Wirkstoffzubereitung der gewünschten Konzentration

behandelt. Ein Blatt der behandelten Pflanze wird in eine Plastikdose gelegt und mit Larven ($L_3$) des Meerrettichkäfers (Phaedon cochleariae) besetzt. Nach 2 bis 4 Tagen wird jeweils ein weiteres Blatt von derselben Pflanze für die Nachfütterung verwendet.

Nach der gewünschten Zeit wird die Abtötung in % bestimmt. Dabei bedeutet 100 %, daß alle Tiere abgetötet wurden; 0 % bedeutet, daß keine tiere abgetötet wurden.

Bei diesem Test zeigten bei einer beispielhaften Wirkstoffkonzentration von 0,1 % nach 14 Tagen z.B. die Verbindungen der Herstellungsbeispiele (1), (5) und (21) eine Wirkung von 100 %, während die Vergleichsverbindungen (A), (B) und (C) eine Wirkung von 10 %, 20 % bzw. 0 % Wirkung aufwiesen.

## Patentansprüche

**1.** Substituierte Furazane der Formel (I)

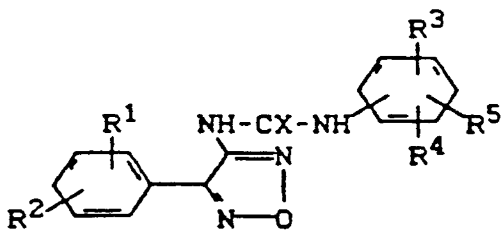

(I)

in welcher

| | |
|---|---|
| $R^1$ und $R^2$ | gleich oder verschieden sind und für Wasserstoff, Halogen, $C_1$-$C_6$-Alkyl, $C_1$-$C_6$-Alkoxy, $C_1$-$C_6$-Alkylthio, Halogen-$C_1$-$C_6$-alkyl, Halogen-$C_1$-$C_6$-alkoxy, Halogen-$C_1$-$C_6$-alkylthio oder für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy und/oder Halogen-$C_1$-$C_4$-alkylthio substituiertes Aryloxy mit 6 bis 10 Kohlenstoffatomen im Arylteil stehen, oder |
| $R^1$ und $R^2$ | gemeinsam für einen gegebenenfalls durch Halogen und/oder $C_1$-$C_2$-Alkyl substituierten $C_1$-$C_3$-Alkylen-Rest, welcher durch 1 oder 2 Sauerstoffatome unterbrochen wird oder über 1 oder 2 Sauerstoffatome an dem Phenylring gebunden ist, stehen, |
| $R^3$ und $R^4$ | gleich oder verschieden sind und für Wasserstoff, Halogen, $C_1$-$C_6$-Alkyl, $C_1$-$C_6$-Alkoxy, Halogen-$C_1$-$C_6$-alkyl oder Halogen-$C_1$-$C_4$-alkoxy stehen, |
| $R^5$ | für gegebenenfalls durch Halogen und/oder $C_1$-$C_2$-Alkyl substituiertes Cycloalkyl mit 3 bis 8 Kohlenstoffatomen steht und |
| X | für Sauerstoff oder Schwefel steht, |

**2.** Substituierte Furazane der Formel (I) gemäß Anspruch 1, in welcher

| | |
|---|---|
| $R^1$ und $R^2$ | gleich oder verschieden sind und für Wasserstoff, Fluor, Chlor, Brom, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy, Halogen-$C_1$-$C_4$-alkylthio oder für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy und/oder Halogen-$C_1$-$C_4$-alkylthio substituiertes Phenoxy stehen, oder |
| $R^1$ und $R^2$ | gemeinsam für einen gegebenenfalls durch Fluor, Chlor und/oder Methyl substituierten $C_1$-$C_3$-Alkylen-Rest, welcher durch 1 oder 2 Sauerstoffatome unterbrochen wird oder über 1 oder 2 Sauerstoffatome an den Phenylring gebunden ist, stehen, |
| $R^3$ und $R^4$ | gleich oder verschieden sind und für Wasserstoff, Fluor, Chlor, Brom, $C_1$-$C_4$-Alkyl, $C_1$-$C_4$-Alkoxy, Halogen-$C_1$-$C_4$-alkyl oder Halogen-$C_1$-$C_4$-alkoxy stehen, |
| $R^5$ | für gegebenenfalls durch Fluor, Chlor, Brom und/oder Methyl substituiertes Cycloalkyl mit 3 bis 6 Kohlenstoffatomen steht und |
| X | für Sauerstoff oder Schwefel steht. |

**3.** Substituierte Furazane der Formel (I) gemäß Anspruch 1, in welcher

| | |
|---|---|
| $R^1$ und $R^2$ | gleich oder verschieden sind und für Wasserstoff, Fluor oder Chlor stehen, |
| $R^2$ und $R^3$ | für Wasserstoff stehen, |
| $R^5$ | für 2-Chlor-2,3,3-trifluor-cyclobutyl, 1-Methyl-2-chlor-2,3,3-trifluorcyclobutyl oder |

2,2,3,3-Tetrafluor-cyclobutyl stehen und
X für Sauerstoff oder Schwefel stehen.

**4.** Verfahren zur Herstellung der substituierten Furazane der Formel (I)

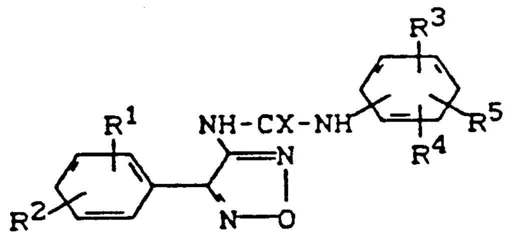

(I)

in welcher
$R^1$ bis $R^5$ und X die in Anspruch 1 angegebenen Bedeutungen haben,
dadurch gekennzeichnet, daß man
a) 3-Amino-1,2,5-oxadiazole der Formel (II)

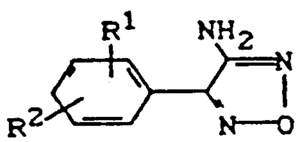

(II)

in welcher
$R^1$ und $R^2$ die oben angegebenen Bedeutungen haben,
mit Iso(thio)cyanaten der Formel (III)

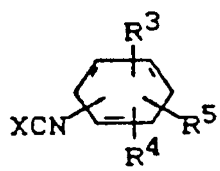

(III)

in welcher
X, $R^3$, $R^4$ und $R^5$ die oben angegebenen Bedeutungen haben,
gegebenenfalls in Gegenwart von Katalysatoren und gegebenenfalls in Gegenwart von Verdünnungsmitteln bei Temperaturen zwischen 20 und 180 °C umsetzt,
oder
b) 3-Iso(thio)cyanato-1,2,5-oxadiazole der Formel (IV)

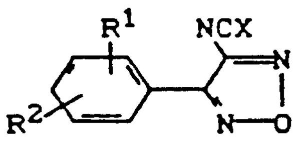

(IV)

in welcher
X, $R^1$ und $R^2$ die oben angegebenen Bedeutungen haben,
mit Anilinen der Formel (V)

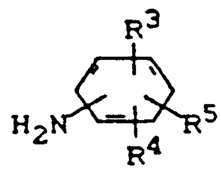

(V)

in welcher
$R^3$, $R^4$ und $R^5$ die oben angegebenen Bedeutungen haben,
gegebenenfalls in Gegenwart von Katalysatoren und gegebenenfalls in Gegenwart von Verdünnungsmitteln bei Temperaturen zwischen 20 und 200 °C umsetzt.

**5.** Schädlingsbekämpfungsmittel, gekennzeichnet durch einen Gehalt an mindestens einer Verbindung gemäß den Ansprüchen 1 bis 3.

**6.** Verwendung von Verbindungen gemäß Ansprüchen 1 bis 3 zur Bekämpfung von Schädlingen, insbesondere Insekten und Spinnentieren.

**7.** Verfahren zur Bekämpfung von Schädlingen, dadurch gekennzeichnet, daß man Verbindungen gemäß den Ansprüchen 1 bis 3 auf Schädlinge, insbesondere Insekten oder Spinnentiere oder ihren Lebensraum einwirken läßt.

**8.** Verfahren zur Herstellung von Schädlingsbekämpfungsmitteln, dadurch gekennzeichnet, daß man Verbindungen gemäß den Ansprüchen 1 bis 3 mit Streckmitteln und/oder oberflächenaktiven Mitteln vermischt.

**9.** Verbindungen der Formel (VI)

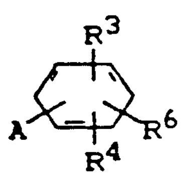

(VI)

in welcher
A für $-NH_2$ oder -NCX steht, wobei
X für Sauerstoff oder Schwefel steht,
$R^3$ und $R^4$ gleich oder verschieden sind und für Wasserstoff, Halogen, $C_{1-6}$ Alkyl, $C_{1-6}$ Alkoxy, Halogen $C_{1-6}$ alkyl oder Halogen $C_{1-4}$ alkoxy stehen und
$R^6$ für 2,2-Difluor-1-methyl-cycloprop-1-yl oder für den Rest

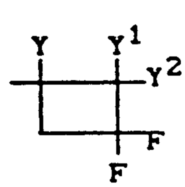

steht, worin
Y für Wasserstoff, Methyl, Fluor oder Chlor steht und
$Y^1$ und $Y^2$ gleich oder verschieden sind und für Fluor oder Chlor stehen,
ausgenommen die Verbindungen in welchen $R^3$ und $R^4$ für H stehen, A für $NH_2$ oder NCO steht und $R^6$ für 2,2,3,3-Tetrafluorocyclobutyl steht.

**10.** Verfahren zur Herstellung der Verbindungen der Formel (VI)

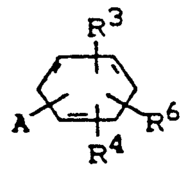

(VI)

in welcher

A, $R^3$, $R^4$ und $R^6$ die in Anspruch 9 angegebenen Bedeutungen haben,

dadurch gekennzeichnet, daß man Nitrobenzole der Formel (VII)

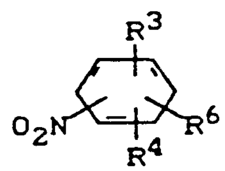

(VII)

in welcher

$R^3$, $R^4$ und $R^6$ die oben angegebene Bedeutung haben,

in üblicher Weise reduziert, vorzugsweise durch katalytische Hydrierung oder mit Hilfe von Metallen oder Metallsalzen und gegebenenfalls die entstandene Aminoverbindung mit Phosgen oder Thiophosgen umsetzt.

**11.** Nitrobenzole der Formel (VII)

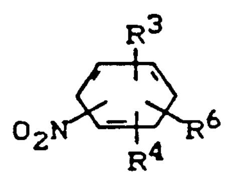

(VII)

in welcher

$R^3$ und $R^4$ gleich oder verschieden sind und für Wasserstoff, Halogen, $C_{1-6}$ Alkyl, $C_{1-6}$ Alkoxy, Halogen $C_{1-6}$ alkyl oder Halogen $C_{1-4}$ alkoxy stehen und

$R^6$ für 2,2-Difluor-1-methyl-cycloprop-1-yl oder für den Rest

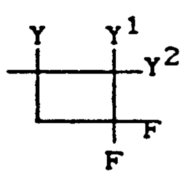

steht, worin

Y für Wasserstoff, Methyl, Fluor oder Chlor steht und

$Y^1$ und $Y^2$ gleich oder verschieden sind und für Fluor oder Chlor stehen.

**12.** Verfahren zur Herstellung der Nitrobenzole der Formel (VII)

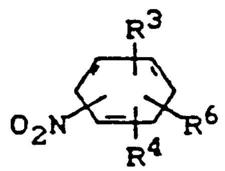

(VII)

in welcher

$R^3$, $R^4$ und $R^6$ die in Anspruch 11 angegebenen Bedeutunten haben,

dadurch gekennzeichnet, daß man die Verbindungen der Formel (VIII)

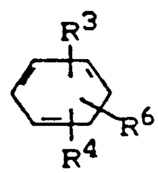

(VIII)

in welcher

$R^3$, $R^4$ und $R^6$ die oben angegebene Bedeutung haben,

nach allgemein üblichen Methoden der Kernnitrierung, vorzugsweise durch Umsetzung mit Nitriersäure ($HNO_3/H_2SO_4$), nitriert.

## Claims

**1.** Substituted furazans of the formula (I)

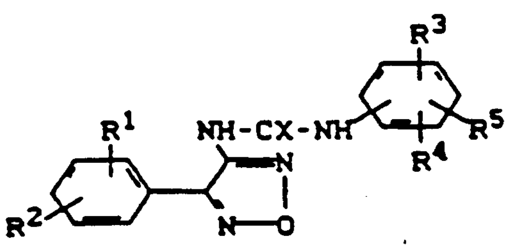

(I)

in which

| | |
|---|---|
| $R^1$ and $R^2$ | are identical or different and represent hydrogen, halogen, $C_1$-$C_6$-alkyl, $C_1$-$C_6$-alkoxy, $C_1$-$C_6$-alkylthio, halogeno-$C_1$-$C_6$-alkyl, halogeno-$C_1$-$C_6$-alkoxy, halogeno-$C_1$-$C_6$-alkylthio or aryloxy, having 6 to 10 carbon atoms in the aryl part, which is optionally substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy and/or halogeno-$C_1$-$C_4$-alkylthio, or |
| $R^1$ and $R^2$ | together represent a $C_1$-$C_3$-alkylene radical which is interrupted by 1 or 2 oxygen atoms or bonded to the phenyl ring via 1 or 2 oxygen atoms and which is optionally substituted by halogen and/or $C_1$-$C_2$-alkyl, |
| $R^3$ and $R^4$ | are identical or different and represent hydrogen, halogen, $C_1$-$C_6$-alkyl, $C_1$-$C_6$-alkoxy, halogeno-$C_1$-$C_6$-alkyl or halogeno-$C_1$-$C_4$-alkoxy, |
| $R^5$ | represents cycloalkyl, having 3 to 8 carbon atoms, which is optionally substituted by halogen and/or $C_1$-$C_2$-alkyl, and |
| X | represents oxygen or sulphur. |

**2.** Substituted furazans of the formula (I) according to Claim 1, in which

$R^1$ and $R^2$ are identical or different and represent hydrogen, fluorine, chlorine, bromine, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy, halogeno-$C_1$-$C_4$-alkylthio or phenoxy which is optionally substituted by halogen, nitro,

cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy and/or halo-geno-$C_1$-$C_4$-alkylthio, or

$R^1$ and $R^2$ together represent a $C_1$-$C_3$-alkylene radical which is interrupted by 1 or 2 oxygen atoms or bonded to the phenyl ring via 1 or 2 oxygen atoms and which is optionally substituted by fluorine, chlorine and/or methyl,

$R^3$ and $R^4$ are identical or different and represent hydrogen, fluorine, chlorine, bromine, $C_1$-$C_4$-alkyl, $C_1$-$C_4$-alkoxy, halogeno-$C_1$-$C_4$-alkyl or halogeno-$C_1$-$C_4$-alkoxy,

$R^5$ represents cycloalkyl, having 3 to 6 carbon atoms, which is optionally substituted by fluorine, chlorine, bromine, and/or methyl, and

X represents oxygen or sulphur.

**3.** Substituted furazans of the formula (I) according to Claim 1, in which

$R^1$ and $R^2$ are identical or different and represent hydrogen, fluorine or chlorine,

$R^2$ and $R^3$ represent hydrogen,

$R^5$ represents 2-chloro-2,3,3-trifluoro-cyclobutyl, or 1-methyl-2-chloro-2,3,3-trifluoro-cyclobutyl or 2,2,3,3,-tetrafluoro-cyclobutyl, and

X represents oxygen or sulphur.

**4.** Process for the preparation of the substituted furazans of the formula (I)

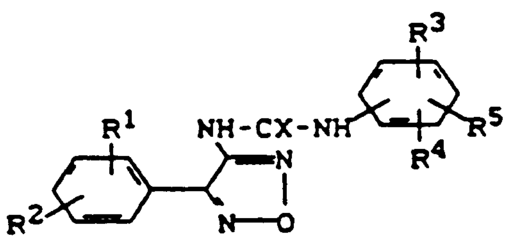

(I)

in which

$R^1$ to $R^5$ and X have the meanings given in Claim 1, characterised in that

a) 3-amino-1,2,5-oxadiazoles of the formula (II) in which

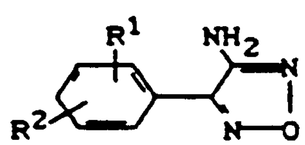

(II)

$R^1$ and $R^2$ have the abovementioned meanings, are reacted at temperatures between 20 and 180 ° C with iso(thio)cyanates of the formula (III)

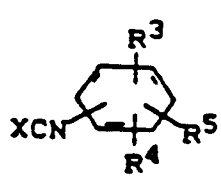

(III)

in which

X, $R^3$, $R^4$ and $R^5$ have the abovementioned meanings,

if appropriate in the presence of catalysts and if appropriate in the presence of diluents,

or

b) 3-iso(thio)cyanato-1,2,5-oxadiazoles of the formula (IV)

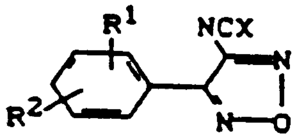

(IV)

in which

X, $R^1$ and $R^2$ have the abovementioned meanings, are reacted at temperatures between 20 and 200 ° C with anilines of the formula (V)

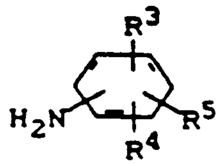

(V)

in which

$R^3$, $R^4$ and $R^5$ have the abovementioned meanings,

if appropriate in the presence of catalysts and if appropriate in the presence of diluents.

**5.** Pesticides, characterised in that they contain at least one compound according to Claims 1 to 3.

**6.** Use of compounds according to Claims 1 to 3 for combating pests, particularly insects and arachnida.

**7.** Process for combating pests, characterised in that compounds according to Claims 1 to 3 are allowed to act on pests, particularly insects or arachnida, or on their habitat.

**8.** Process for the preparation of pesticides, characterised in that compounds according to Claims 1 to 3 are mixed with extenders and/or surface-active agents.

**9.** Compounds of the formula (VI)

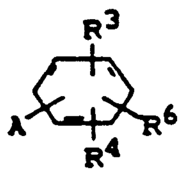

(VI)

in which

A represents $-NH_2$ or -NCX, where

X represents oxygen or sulphur,

$R^3$ and $R^4$ are identical or different and represent hydrogen, halogen, $C_1$-$C_6$-alkyl, $C_1$-$C_6$-alkoxy, halogen-$C_1$-$C_6$-alkyl or halogeno-$C_1$-$C_4$-alkoxy, and

$R^6$ represents 2,2-difluoro-1-methyl-cycloprop-1-yl or the

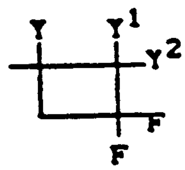

radical, in which

Y represents hydrogen, methyl, fluorine or chlorine, and
$Y^1$ and $Y^2$ are identical or different and represent fluorine or chlorine, except for the compounds in which $R^3$ and $R^4$ represent H, A represents $NH_2$ or NCO and $R^6$ represents 2,2,3,3-tetrafluoro-cyclobutyl.

**10.** Process for the preparation of the compounds of the formula (VI)

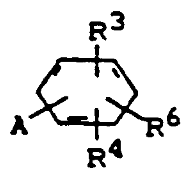

(VI)

in which
A, $R^3$, $R^4$ and $R^6$ have the meanings given in Claim 9,
characterised in that nitrobenzenes of the formula (VII)

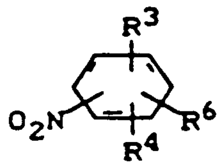

(VII)

in which
$R^3$, $R^4$ and $R^6$ have the abovementioned meaning,
are reduced in a conventional fashion, preferably by catalytic hydrogenation or with the aid of metals or metal salts, and, if appropriate, the resultant amino compound is reacted with phosgene or thiophosgene.

**11.** Nitrobenzenes of the formula (VII)

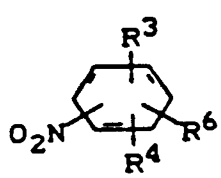

(VII)

in which
$R^3$ and $R^4$ are identical or different and represent hydrogen, halogen, $C_1$-$C_6$-alkyl, $C_1$-$C_6$-alkoxy, halogeno-$C_1$-$C_6$-alkyl or halogeno-$C_1$-$C_4$-alkoxy, and
$R^6$ represents 2,2-difluoro-1-methyl-cycloprop-1-yl or the

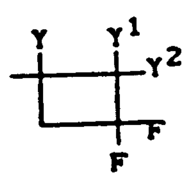

radical, in which

| | |
|---|---|
| Y | represents hydrogen, methyl, fluorine or chlorine, and |
| $Y^1$ and $Y^2$ | are identical or different and represent fluorine or chlorine. |

**12.** Process for the preparation of the nitrobenzenes of the formula (VII)

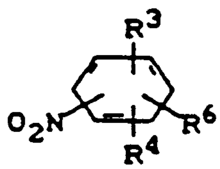

(VII)

in which

| | |
|---|---|
| $R^3$, $R^4$ and $R^6$ | have the meanings given in Claim 11, characterised in that the compounds of the formula (VIII) |

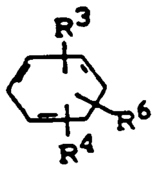

(VIII)

in which

| | |
|---|---|
| $R^3$, $R^4$ and $R^6$ | have the abovementioned meaning, |

are nitrated by generally conventional methods of nuclear nitration, preferably by reaction with nitrating acid ($HNO_3/H_2SO_4$).

## Revendications

**1.** Furazanes substitués de la formule (I)

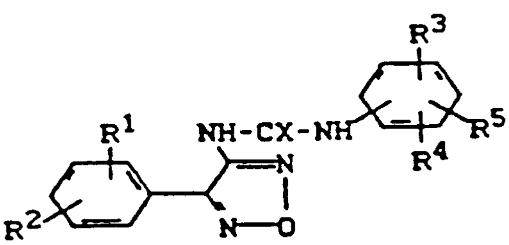

(I)

dans laquelle

| | |
|---|---|
| $R^1$ et $R^2$ | sont identiques ou différents et représentent l'hydrogène, un halogène, un alkyle de $C_1$ à $C_6$, alcoxy de $C_1$ à $C_6$, alkylthio de $C_1$ à $C_6$, un halogènalkyle de $C_1$ à $C_6$, halogènalcoxy de $C_1$ à $C_6$, halogènalkylthio de $C_1$ à $C_6$ ou un aryloxy dont la partie aryle comporte 6 à 10 atomes de carbone, substitué le cas échéant par un halogène, nitro, cyano, alkyle de $C_1$ à $C_4$, alcoxy de $C_1$ à $C_4$, alkylthio de $C_1$ à $C_4$, halogènalkyle de $C_1$ à $C_4$, halogènalcoxy de $C_1$ à $C_4$, et/ou halogènalkylthio de $C_1$ à $C_4$, ou |
| $R^1$ et $R^2$ | représentent conjointement un radical alkylène de $C_1$ à $C_3$ éventuellement substitué par un halogène et/ou par un alkyle de $C_1$ à $C_2$, qui est interrompu par 1 ou par 2 atomes d'oxygène ou lié au cycle phényle par 1 ou par 2 atomes d'oxygène, |
| $R^3$ et $R^4$ | sont identiques ou différents et représentent l'hydrogène, un halogène, alkyle de $C_1$ à $C_6$, alcoxy de $C_1$ à $C_6$, halogènalkyle de $C_1$ à $C_6$, ou halogènalcoxy de $C_1$ à $C_4$, |
| $R^5$ | représente un cycloalkyle comportant 3 à 8 atomes de carbone, éventuellement substitué par un halogène et/ou par un alkyle de $C_1$ à $C_2$ et |

X représente l'oxygène ou le soufre.

**2.** Furazanes substitués de la formule (I) selon la revendication 1, dans laquelle

$R^1$ et $R^2$ sont identiques ou différents et représentent l'hydrogène, le fluor, le chlore, le brome, un alkyle de $C_1$ à $C_4$, un alcoxy de $C_1$ à $C_4$, un alkylthio de $C_1$ à $C_4$, un halogènalkyle de $C_1$ à $C_4$, un halogènalcoxy de $C_1$ à $C_4$, un halogènalkylthio de $C_1$ à $C_4$ ou un phénoxy substitué éventuellement par un halogène, nitro, cyano, alkyle de $C_1$ à $C_4$, alcoxy de $C_1$ à $C_4$, alkylthio de $C_1$ à $C_4$, halogènalkyle de $C_1$ à $C_4$, halogènalcoxy de $C_1$ à $C_4$, et/ou halogènalkylthio de $C_1$ à $C_4$, ou

$R^1$ et $R^2$ représentent conjointement un radical alkylène de $C_1$ à $C_3$ éventuellement substitué par le fluor, le chlore et/ou un méthyle, qui est interrompu par 1 ou par 2 atomes d'oxygène ou lié au cycle phényle par 1 ou par 2 atomes d'oxygène,

$R^3$ et $R^4$ sont identiques ou différents et représentent l'hydrogène, le fluor, le chlore, le brome, un alkyle de $C_1$ à $C_4$, un alcoxy de $C_1$ à $C_4$, un halogènalkyle de $C_1$ à $C_4$, ou un halogènalcoxy de $C_1$ à $C_4$,

$R^5$ représente un cycloalkyle avec 3 à 6 atomes de carbone, éventuellement substitué par le fluor, le chlore, le brome et/ou un méthyle et

X représente l'oxygène ou le soufre.

**3.** Furazanes substitués de la formule (I) selon la revendication 1, dans laquelle

$R^1$ et $R^2$ sont identiques ou différents et représentent l'hydrogène, le fluor ou le chlore,

$R^2$ et $R^3$ correspondent à l'hydrogène,

$R^5$ représente le 2-chloro-2,3,3-trifluoro-cyclobutyle, le 1-méthyl-2-chloro-2,3,3-trifluoro-cyclobutyle ou le 2,2,3,3-tétrafluoro-cyclobutyle et

X est l'oxygène ou le soufre.

**4.** Procédé de préparation des furazanes substitués de la formule (I)

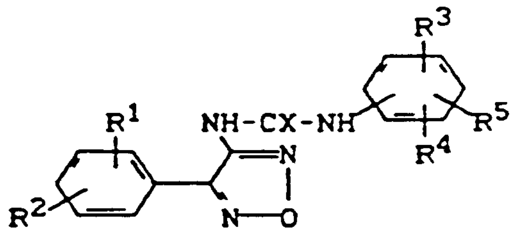

(I)

dans laquelle

$R^1$ à $R^5$ possèdent la signification indiquée dans la revendication 1,

caractérisé en ce que l'on fait réagir des

a) des 3-amino-1,2,5-oxadiazols de la formule (II)

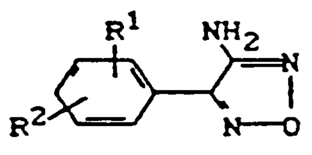

(II)

dans laquelle

$R^1$ et $R^2$ possèdent la signification indiquée ci-dessus, avec des iso(thio)cyanates de la formule (III)

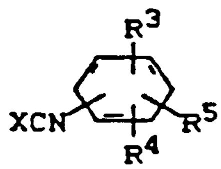

(III)

dans laquelle
X, $R^3$, $R^4$ et $R^5$ possèdent la signification indiquée ci-dessus,
le cas échéant, en présence de catalyseurs et éventuellement en présence de diluants, à des températures comprises entre 20 et 180 °C,
ou en faisant réagir
b) des 3-iso(thio)cyanato-1,2,5-oxadiazols de la formule (IV)

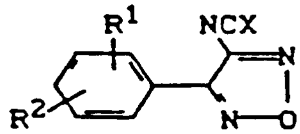

(IV)

dans laquelle
X, $R^1$ et $R^2$ possèdent la signification indiquée ci-dessus, avec des anilines de la formule (V)

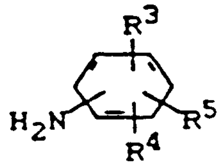

(V)

dans laquelle
$R^3$, $R^4$ et $R^5$ possèdent la signification indiquée ci-dessus,
le cas échéant, en présence de catalyseurs et éventuellement en présence de diluants, à des températures comprises entre 20 et 200 °C.

**5.** Agents de lutte contre les parasites, caractérisés par une teneur en au moins un composé selon les revendications 1 à 3.

**6.** Mise en oeuvre des composés selon les revendications 1 à 3 pour la lutte contre les parasites, en particulier les insectes et les arachnidés.

**7.** Méthode de lutte contre les parasites, caractérisée en ce que l'on fait agir des composés selon les revendications 1 à 3 sur des parasites, en particulier des insectes ou des arachnidés, ou sur leur espace vital.

**8.** Procédé de préparation des agents de lutte contre les parasites, caractérisé en ce que l'on mélange des composés selon les revendications 1 à 3 avec des diluants et/ou avec des agents tensioactifs.

**9.** Composés de la formule (VI)

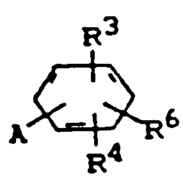

(VI)

dans laquelle

| | |
|---|---|
| A | représente un radical $-NH_2$ ou -NCX, dans lequel X est l'oxygène ou le soufre, |
| $R^3$ et $R^4$ | sont identiques ou différents et représentent l'hydrogène, un halogène, un alkyle de $C_1$ à $C_6$, un alcoxy de $C_1$ à $C_6$, un halogènalkyle de $C_1$ à $C_6$ ou un halogènalcoxy de $C_1$ à $C_4$ et |
| $R^6$ | représente le 2,2-difluoro-1-méthyl-cycloprop-1-yle ou le radical |

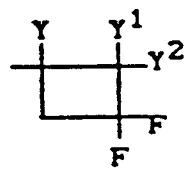

,

dans lequel
Y représente l'hydrogène, un méthyle, le fluor ou le chlore et
$Y^1$ et $Y^2$ sont identiques ou différents et représentent le fluor ou le chlore, excepté les composés dans lesquels $R^3$ et $R^4$ représentent l'hydrogène, A correspond à $NH_2$ ou à NCO et $R^6$ représente le 2,2,3,3-tétrafluoro-cyclobutyle.

**10.** Procédé de préparation des composés de la formule (VI)

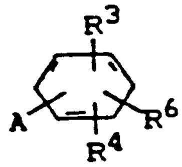

(VI)

dans laquelle
A, $R^3$, $R^4$ et $R^6$ possèdent la signification indiquée dans la revendication 9,
caractérisé en ce que l'on réduit des nitrobenzènes de la formule (VII)

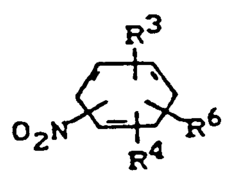

(VII)

dans laquelle
$R^3$, $R^4$ et $R^6$ possèdent la signification indiquée ci-dessus, selon une méthode usuelle, de préférence par hydrogénation catalytique ou à l'aide de métaux ou de sels métalliques et, en ce que l'on fait réagir, le cas échéant, le composé amino formé avec du phosgène ou du thiophosgène.

**11.** Nitrobenzènes de la formule (VII)

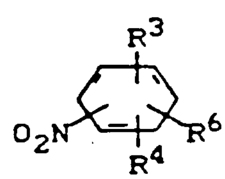

(VII)

dans laquelle

$R^3$ et $R^4$ sont identiques ou différents et représentent l'hydrogène, un halogène, un alkyle de $C_1$ à $C_6$, un alcoxy de $C_1$ à $C_6$, un halogènalkyle de $C_1$ à $C_6$ ou un halogènalcoxy de $C_1$ à $C_4$ et

$R^6$ représente le 2,2-difluoro-1-méthyl-cycloprop-1-yle ou le radical

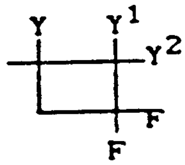

,

dans lequel
Y représente l'hydrogène, un méthyle, le fluor ou le chlore et
$Y^1$ et $Y^2$ sont identiques ou différents et représentent le fluor ou le chlore.

**12.** Procédé de préparation des nitrobenzènes de la formule (VII)

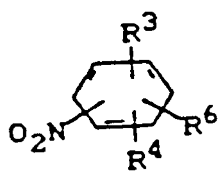

(VII)

dans laquelle
$R^3$, $R^4$ et $R^6$ possèdent la signification indiquée dans la revendication 11,
caractérisé en ce que l'on nitrifie les composés de la formule (VIII)

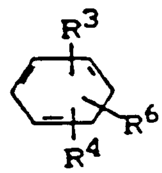

dans laquelle
$R^3$, $R^4$ et $R^6$ possèdent la signification indiquée ci-dessus, selon les méthodes usuelles de la nitrification sur le noyau, de préférence par mise en réaction avec le mélange sulfonitrique ($HNO_3/H_2SO_4$).